# EUROPEAN PATENT SPECIFICATION

(11) **EP 1 903 965 B1**
(45) Date of publication and mention of the grant of the patent: **07.07.2010**
(21) Application number: 06773554.8
(22) Date of filing: 20.06.2006
(51) Int. Cl.: A61B 17/70

(54) **INTERVERTEBRAL PROSTHETIC DEVICE FOR SPINAL STABILIZATION**
BANDSCHEIBENPROTHESE ZUR WIRBELSÄULENSTABILISIERUNG
DISPOSITIF PROTHETIQUE INTERVERTEBRAL POUR STABILISATION VERTEBRALE

(30) Priority: 27.06.2005 US 167775
(43) Date of publication of application: 02.04.2008
(73) Proprietor: Warsaw Orthopedic, Inc., Warsaw, IN 46581 (US)
(72) Inventor: BRUNEAU, Aurelien, Memphis, Tennessee 38016 (US); CARLS, Thomas, A., Memphis, Tennessee 38103 (US); LANGE, Eric, C., Collierville, Tennessee 38017 (US); MOLZ, Fred, J. IV, Birmingham, Alabama 35242 (US); MORRISON, Matthew, M., Cordova, Tennessee 38018 (US); DEWEY, Jonathan, M., Memphis, Tennessee 38103 (US); ANDERSON, Kent, M., Sanford, CA 94305 (US)
(74) Representative: Price, Nigel John King
(86) International application number: PCT/US2006/023831
(87) International publication number: WO 2007/001994

(56) References cited:
- WO-A-2005/009300
- US-A- 5 496 318
- US-A- 5 836 948

## Description

### Background

The present invention relates to an intervertebral prosthetic device for stabilizing the human spine.

Spinal discs that extend between adjacent vertebrae in vertebral columns of the human body provide critical support between the adjacent vertebrae. These discs can rupture, degenerate, and/or protrude by injury, degradation, disease, or the like to such a degree that the intervertebral space between adjacent vertebrae collapses as the disc loses at least a part of its support function, which can cause impingement of the nerve roots and severe pain.

In these cases, intervertebral prosthetic devices have been designed that can be implanted between the adjacent vertebrae, both anterior and posterior of the column, to prevent the collapse of the intervertebral space between the adjacent vertebrae and thus stabilize the spine.

However, many of these devices are relatively stiff, and, as such, cannot flex to better accommodate the vertebrae and do not provide a sufficient amount of deformability. Also, many of these devices, when implanted, suffer from a relatively high fatigue.

WO2005/009300A, disclosing a prosthesis for insertion between the spinous processes of the vertebra, and US-A05836948, disclosing a spine distraction implant and method, provide background art to the present invention.

### Summary

The intervertebral prosthetic device according to the invention is as defined by the claims. Embodiments of the invention overcome the above deficiencies by providing relatively low fatigue characteristics and relatively high deformability, resulting in a good fit with the anatomy.
Various embodiments of the invention may possess one or more of the above features and advantages, or provide one or more solutions to the above problems existing in the prior art.

### Brief Description of the Drawings

Fig. 1 is a side elevational view of an adult human vertebral column.
Fig. 2 is a posterior elevational view of the column of Fig. 1.
Fig. 3 is an enlarged, front elevational, view of one of the vertebrae of the column of Figs. 1 and 2.
Fig. 4 is an enlarged, partial, isometric view of a portion of the column of Figs. 1 and 2, including the lower three vertebrae of the column, and depicting intervertebral prosthetic device according to an embodiment of the invention inserted between two adjacent vertebrae.
Fig. 5 is an enlarged, isometric, exploded view of the prosthetic device of Fig. 3.
Figs. 6 and 7 are enlarged, isometric, exploded views of alternate embodiments of the prosthetic device of Fig. 5.

### Detailed Description

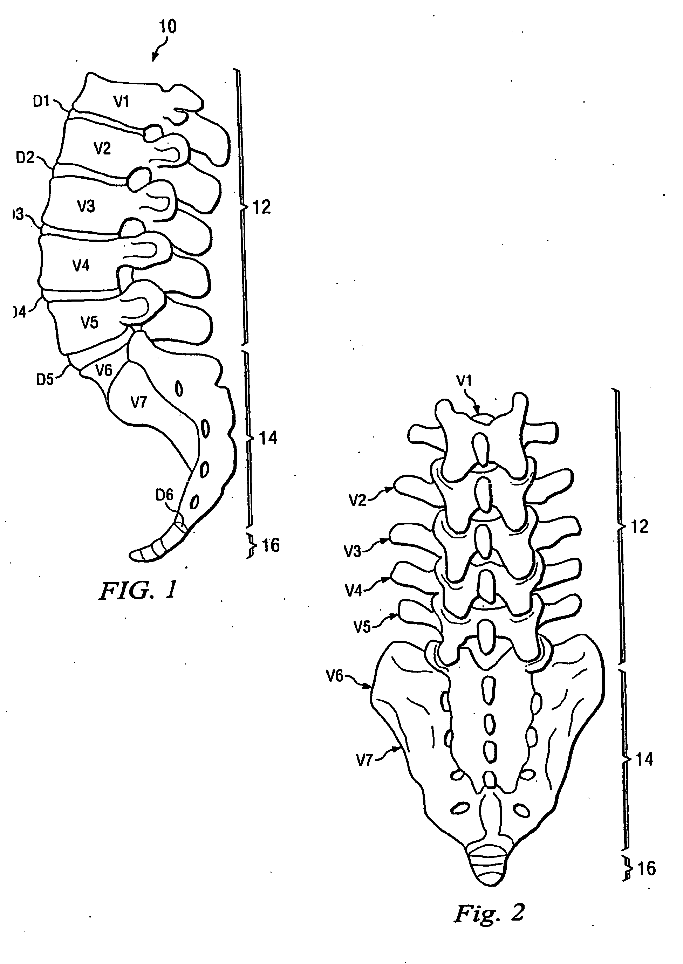

With reference to Figs. 1 and 2, the reference numeral 10 refers, in general, to a human vertebral column 10. The lower portion of the vertebral column 10 is shown and includes the lumbar region 12, the sacrum 14, and the coccyx 16. The flexible, soft portion of the vertebral column 10, which includes the thoracic region and the cervical region, is not shown.

The lumbar region 12 of the vertebral column 10 includes five vertebrae V1, V2, V3, V4 and V5 separated by intervertebral discs D1, D2, D3, and D4, with the disc D1 extending between the vertebrae V1 and V2, the disc D2 extending between the vertebrae V2 and V3, the disc D3 extending between the vertebrae V3 and V4, and the disc D4 extending between the vertebrae V4 and V5.

The sacrum 14 includes five fused vertebrae, one of which is a superior vertebrae V6 separated from the vertebrae V5 by a disc D5. The other four fused vertebrae of the sacrum 14 are referred to collectively as V7. A disc D6 separates the sacrum 14 from the coccyx 16 which includes four fused vertebrae (not referenced).

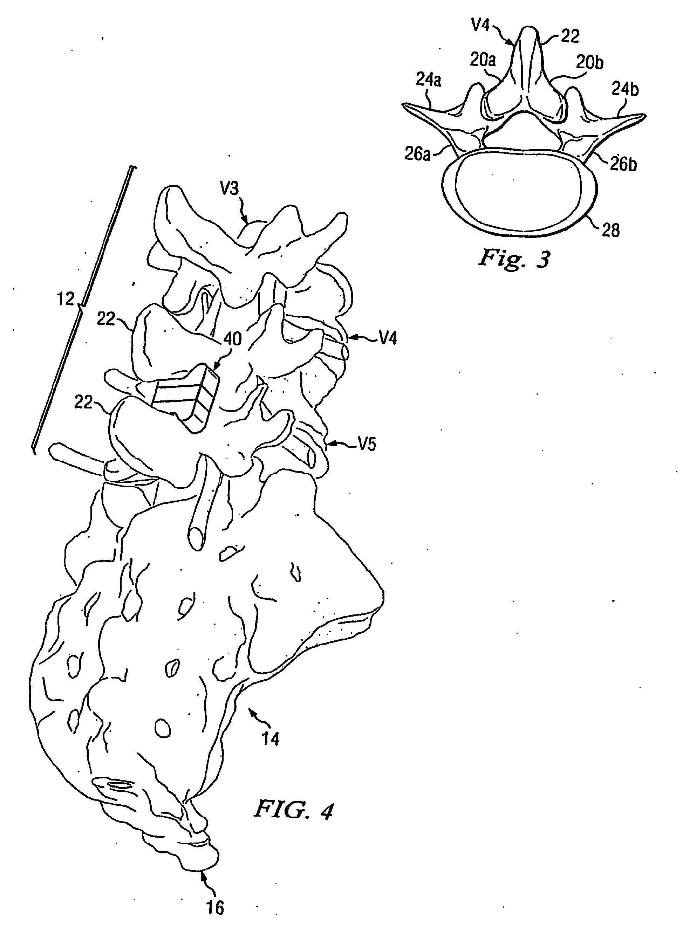

With reference to Fig. 3, the vertebrae V4 includes two laminae 20a and 20b extending to either side (as viewed in Fig. 2) of a spinous process 22 that projects posteriorly from the juncture of the two laminae. Two transverse processes 24a and 24b extend laterally from the laminae 20a and 20b, respectively, and two pedicles 26a and 26b extend inferiorly from the processes 24a and 24b to a vertebral body 28. Since the other vertebrae V1-V3 and V5 are similar to the vertebrae V4, they will not be described in detail.

Referring to Fig. 4, it will be assumed that, for one or more of the reasons set forth above, the vertebrae V4 and V5 are not being adequately supported by the disc D4 and that it is therefore necessary to provide supplemental support and stabilization of these vertebrae. To this end, an intervertebral disc prosthetic device 40 according to an embodiment of the invention is implanted between the spinous processes 22 of the vertebrae V4 and V5.

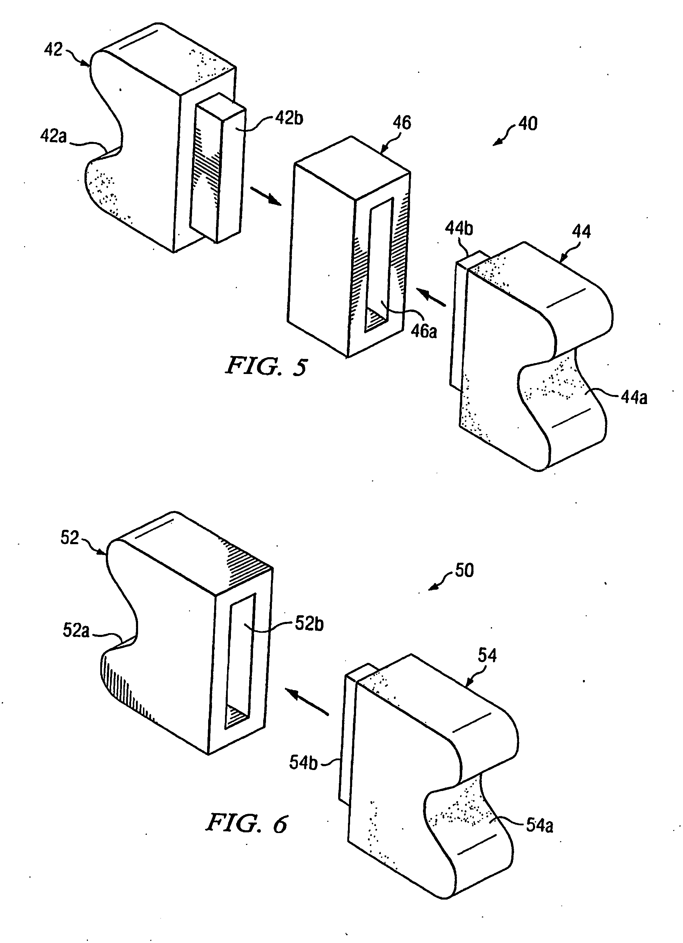

The device 40 is shown in detail in Fig. 5 and includes a body member 42 which is substantially rectangular in shape with the exception that a curved notch 42a, is formed in one end portion. A body member 44 is also provided which is substantially rectangular in shape with the exception that a curved notch 44a, is formed in one end portion.

A mechanism is provided to connect the body members, and includes a connector 46 designed to fit over two connection elements 42b and 44b extending from the body members 42 and 44, respectively. To this end, the connector 46 has a through opening 46a with a cross section slightly greater than the cross sections of the elements 42b and 44b.

To connect the body members 42 and 44, the elements 42b and 44b are inserted into the opening 46a from opposite ends of the opening until the corresponding shoulders of the body members 42 and 44 engage the corresponding ends of the connector 46.

Preferably, the body members 42 and 44 are fabricated from a relatively flexible, soft material and the connecter is fabricated from a relatively stiff material.

When the device 40 is implanted between the two adjacent vertebrae V4 and V5 (Fig. 4), the spinous process 22 of one of the vertebrae V4 or V5 extends in the notch 42a and the spinous process of the other vertebrae extends in the notch 44a, respectively, or vice versa. The relatively flexible, soft body members 42 and 44 provide excellent deformability resulting in an improved fit, and the connector 46 adds stiffness, compressive strength and durability to the device 40.

It is understood that the surgeon could be provided with several connectors 46 that vary in stiffness. Thus, once the surgeon ascertains the condition of the vertebrae V4 and V5 (Fig. 4) and determines the particular stiffness that is needed, the proper connector 46 can be selected.

A prosthetic device 50 according to another embodiment (not forming part of the invention but representing background art useful for understanding the invention) is shown in detail in Fig. 6 and includes a body member 52 which is substantially rectangular in shape with the exception that a curved notch 52a is formed in one end portion. A body member 54 is also provided and is designed to be connected to the body member 52. The body member 54 is substantially rectangular in shape with the exception that a curved notch 54a is formed in one end portion.

A mechanism is provided to connect the body members 52 and 54 and includes a connecting element 54b projecting from the other end of the body member 54 and a rectangularly-shaped opening 52b formed in the other end of the body member 52. The dimensions of the element 54b are slightly less than the corresponding dimensions of the opening 52b. The body members 52 and 54 are connected by inserting the element 54b into the opening 52b until the corresponding faces of the body members abut.

One of the body members 52 and 54 is fabricated from a relatively flexible, soft material and the other body member is fabricated from a relatively stiff material. For the purpose of example, it will be assumed that the body member 52 is fabricated from the relatively, soft flexible material and the body member 54 is fabricated from the relatively stiff material.

When the device 50 is implanted between the two adjacent vertebrae V4 and V5 (Fig. 4), the spinous process 22 of one of the vertebrae V4 or V5 extends in the notch 52a of the body member 52, and the spinous process 22 of the other vertebrae extends in the notch 54a of the body member 54. The relatively flexible, soft, body member 52 provides excellent deformability resulting in an improved fit, and the body member 54 adds stiffness, compressive strength and durability to the device 50.

It is understood that the surgeon could be provided with several body members 54 that vary in stiffness. Thus, once the surgeon ascertains the condition of the vertebrae V4 and V5 (Fig. 4) and determines the particular stiffness that is needed, the proper body member 54 can be selected.

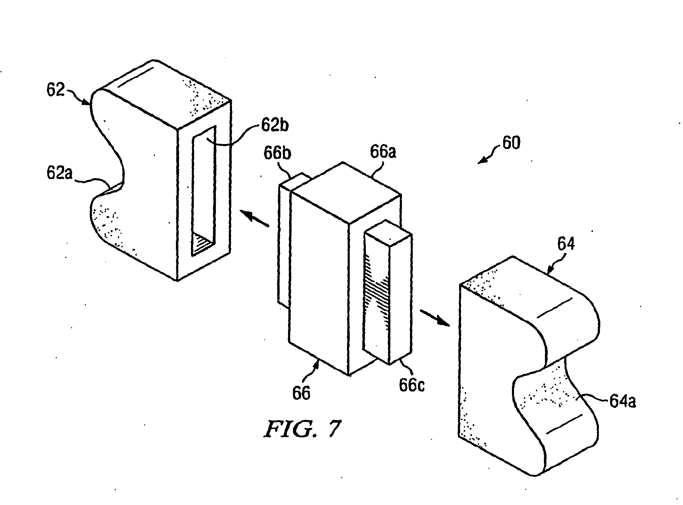

A prosthetic device 60 according to another embodiment is shown in detail in Fig. 7 and includes two body members 62 and 64. The body members 62 and 64 are substantially rectangular in shape with the exception that curved notches 62a and 64a are formed at one end portion of the body members, respectively.

A mechanism 66 is provided for connecting the body members 62 and 64 and includes a rectangular base 66a having a thickness substantially corresponding to the thicknesses of the body members 62 and 64. Two connecting elements 66b and 66c project or extend from opposite faces, respectively, of the base 66.

A rectangularly-shaped opening 62b is formed in the other end of the body member 62 and a rectangularly-shaped opening (not shown) is formed in the other end of the body member 64.

The dimensions of the connecting elements 66b and 66c are slightly less than the dimensions of the opening 62b in the body member 62 and the opening (not shown) in the body member 64.

The element 66b of the connector 66 can thus be inserted in the opening 62b of the body member 62 until the corresponding faces of the connector and the body member abut. Similarly, the element 66c of the connector 66 can be inserted in the above opening of the body member 64 until the corresponding faces of the connector and the body member abut.

The body members 62 and 64 may be fabricated from a relatively flexible, soft material while the connector 66 may be fabricated from a relatively stiff material such as metal or plastic.

When the device 60 is implanted between the two adjacent vertebrae V4 and V5 (Fig. 4), the spinous process 22 of one of the vertebrae V4 or V5 extends in the notch 62a of the body member 62, and the spinous process 22 of the other vertebrae extends in the notch 64a of the body member 64. The relatively flexible, soft body members 62 and 64 provide excellent deformability resulting in an improved fit, and the connector 66 adds stiffness, compressive strength and durability to the device 60.

It is understood that the surgeon could be provided with several mechanisms 66 that vary in hardness and stiffness. Thus, once the surgeon ascertains the condition of the vertebrae V4 and V5 (Fig. 4) and determines the particular stiffness that is needed, the proper mechanism 66 can be selected.

It is understood that, in the embodiments of Figs. 5 and 7, the material making up the body members 42, 44, 62, and 64 can be of a flexible, soft plastic, such as silicon; while the connectors 46 and 66 can be of a relatively stiff rubber, plastic, metal, or other similar material. In the embodiments of Fig. 6, the material making up the body member 52 can be of a flexible, soft plastic, such as silicon and the body member 54 can be of a relatively stiff rubber, plastic, metal, or other similar material; or vice versa.

### Variations

It is understood that variations may be made in the foregoing without departing from the invention and examples of some variations are as follows:

Any conventional substance that promotes bone growth, such as HA coating, BMP, or the like, can be incorporated in each of the above embodiments.

The surfaces of the body members 42, 44, 52, 54, 62, and 64 defining the notches 42a, 44a, 52a, 54a, 62a, and 64a can be treated, such as by providing teeth, ridges, knurling, etc., to better grip the spinous processes.

The body members 42, 44, 52, 54, 62, and 64 can be fabricated of a permanently deformable material thus providing a clamping action against the spinous processes 22.

The relatively stiff components of the above devices may have through holes formed therein to improve integration of the bone growth.

The body members, inserts, and connectors of one or more of the above embodiments may vary in shape, size, composition, and physical properties.

Through openings can be provided through one or more components of each of the above embodiments to receive tethers for attaching the devices to a vertebrae or to a spinous process.

Bilateral extrusions may be provided on the relatively stiff component of each embodiment for tethering the device to a vertebrae or a spinous process.

The relatively stiff components described above could be made of a resorbable material so that their stiffness would change over time.

In the above embodiments, a different mechanism can be provided for connecting the various body members.

The relatively stiff components described above could be replaced by components having a different stiffness pre-operatively or intra-operatively.

In each of the above embodiments, the components that are made of a relatively flexible, soft material could be made of a relatively stiff material and the components that are made of a relatively stiff material could be made of a relatively flexible, soft material.

The prosthetic devices of the above embodiments can be implanted between body portions other than vertebrae.

The prosthetic devices of the above embodiments can be inserted between two vertebrae following a discectemy in which a disc between the adjacent vertebrae is removed, or a corpectomy in which at least one vertebrae is removed.

The prosthetic devices of the above embodiments can be inserted between the facets of adjacent vertebrae, rather than the spinous processes; and

The spatial references made above, such as "under", "over", "between", "lower", "top", "bottom", etc. are for the purpose of illustration only and do not limit the specific orientation or location of the structure described above.

The preceding specific embodiments are illustrative of the practice of the invention. It is to be understood, therefore, that other expedients known to those skilled in the art or disclosed herein, may be employed without departing from the scope of the appended claims, as detailed above.

## Claims

1. A surgical kit for preparing a prosthetic device (40) for insertion between adjacent spinous processes, the kit comprising:
a first member (42) having a notch (42a) formed in an end portion for receiving a first spinous process;
a second member (44) having a notch (44a) formed in an end portion for receiving a second spinous process;
a plurality of connectors (46), with each connector of the plurality having a different stiffness than the other connectors of the plurality,
wherein the first member (42), the second member (44), and any one of the plurality of connectors (46) are connectable to form the prosthetic device such that the notches of the first and second members face away from each other to receive the spinous processes and the connector (46) is positioned between the notches when connected.

2. The surgical kit of claim 1 wherein the first (42) and second members (44) are more flexible than any of the connectors (46).

3. The surgical kit of claim 1 wherein the first member (42) connects to the connector (46) and the connector (46) connects to the second member (44) to form the prosthetic device (40).

4. The surgical kit of claim 3 wherein the first (42) and second (44) members have openings therein for which receive projections from the connectors (46) when connected.

## Patentansprüche

1. Chirurgische Ausrüstung zum Präparieren einer Prothesenvorrichtung (40) zum Einsetzen zwischen Dornfortsätzen, wobei die Ausrüstung umfasst:
ein erstes Element (42) mit einer in einem Endabschnitt ausgebildeten Kerbe (42a), um einen ersten Dornfortsatz aufzunehmen;
ein zweites Element (44) mit einer in einem Endabschnitt ausgebildeten Kerbe (44a), um einen zweiten Dornfortsatz aufzunehmen;
mehrere Verbinder (46), wobei jeder der mehreren Verbinder eine andere Steifigkeit als die anderen der mehreren Verbinder besitzt,
wobei das erste Element (42), das zweite Element (44) und irgendeiner der mehreren Verbinder (46) verbunden werden können, um eine Prothesenvorrichtung zu bilden, derart, dass die Kerben des ersten und des zweiten Elements voneinander weggerichtet sind, um die Dornfortsätze aufzunehmen, und der Verbinder (46) zwischen den Kerben positioniert ist, wenn die Verbindung besteht.

2. Chirurgische Ausrüstung nach Anspruch 1, wobei das erste Element (42) und das zweite Element (44) biegsamer sind als irgendeiner der Verbinder (46).

3. Chirurgische Ausrüstung nach Anspruch 1, wobei das erste Element (42) mit dem Verbinder (46) verbunden ist und der Verbinder (46) mit dem zweiten Element (44) verbunden ist, um die Prothesenvorrichtung (40) zu bilden.

4. Chirurgische Ausrüstung nach Anspruch 3, wobei in dem ersten Element (42) und in dem zweiten Element (44) Öffnungen vorhanden sind, die Vorsprünge der Verbinder (46) aufnehmen, wenn die Verbindung besteht.

## Revendications

1. Sous-ensemble chirurgical pour préparer un dispositif prothétique (40) destiné à être inséré entre des processus épineux adjacents, le sous-ensemble comprenant :
un premier élément (42) ayant une encoche (42a) formée dans une portion terminale pour recevoir un premier processus épineux ; et
un second élément (44) ayant une encoche (44a) formée dans une portion terminale pour recevoir un second processus épineux ;
une pluralité de connecteurs (46), chaque connecteur de la pluralité ayant une raideur différente de celle des autres connecteurs de la pluralité,
dans lequel le premier élément (42), le second élément (44) et l'un quelconque de la pluralité de connecteurs (46) sont susceptibles d'être connectés pour former le dispositif prothétique, de telle façon que les encoches du premier et du second élément sont tournées en éloignement l'une de l'autre pour recevoir les processus épineux, et le connecteur (46) est positionné entre les encoches lorsqu'il est connecté.

2. Sous-ensemble chirurgical selon la revendication 1, dans lequel le premier élément (42) et le second élément (44) sont plus flexibles que l'un quelconque des connecteurs (46).

3. Sous-ensemble chirurgical selon la revendication 1, dans lequel le premier élément (42) est connecté au connecteur (46) et le connecteur (46) est connecté au second élément (44) pour former le dispositif prothétique (40).

4. Sous-ensemble chirurgical selon la revendication 3, dans lequel le premier élément (42) et le second élément (44) comportent des ouvertures en eux-mêmes, qui reçoivent des projections provenant des connecteurs (46) lorsqu'ils sont connectés.
